(19) Europäisches Patentamt
European Patent Office
Office européen des brevets

(11) Veröffentlichungsnummer : **0 140 062**
**B1**

(12) # EUROPÄISCHE PATENTSCHRIFT

(45) Veröffentlichungstag der Patentschrift : **06.05.87**

(21) Anmeldenummer : **84110665.1**

(22) Anmeldetag : **07.09.84**

(51) Int. Cl.⁴ : **C 07 C103/46**, C 07 C103/84, C 07 C102/00, C 07 C127/19, C 07 D251/42, C 07 D251/50, C 07 D251/54, D 06 M 13/34

(54) **Perfluoralkyl-anthranilsäureester, Verfahren zu deren Herstellung und ihre Verwendung als schmutzabweisendes Mittel.**

(30) Priorität : **16.09.83 DE 3333457**

(43) Veröffentlichungstag der Anmeldung : **08.05.85 Patentblatt 85/19**

(45) Bekanntmachung des Hinweises auf die Patenterteilung : **06.05.87 Patentblatt 87/19**

(84) Benannte Vertragsstaaten : **BE CH DE FR GB IT LI NL**

(56) Entgegenhaltungen :
**DE-A- 1 795 446**
**DE-B- 1 017 616**
**GB-A- 1 549 297**
**US-A- 4 347 188**

(73) Patentinhaber : **HOECHST AKTIENGESELLSCHAFT**
**Postfach 80 03 20**
**D-6230 Frankfurt am Main 80 (DE)**

(72) Erfinder : **Schinzel, Erich, Dr.**
**Ostpreussenstrasse 43**
**D-6238 Hofheim am Taunus (DE)**
Erfinder : **Pelster, Manfred**
**Ulmenweg 18a**
**D-6720 Speyer (DE)**

**EP 0 140 062 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Perfluoralkylverbindungen, die sich zur schmutzabweisenden Ausrüstung von Fasern oder Geweben aus synthetischen, halbsynthetischen oder natürlichen Materialien, vorzugsweise aus Polyethylenterephthalat oder Polyamiden eignen.

Verbindungen, die Perfluoralkylreste enthalten sind als schmutzabstoßende Mittel bereits bekannt. So werden in der DE-A 2 628 776 Verbindungen beschrieben, die im wesentlichen aus wenigstens einer fluorierten Verbindung mit wenigstens einem Benzolring bestehen. Ferner kennt man schmutzabstoßende Mittel, die aus polymeren Verbindungen, die fluorierte Gruppen enthalten, aufgebaut sind. Speziell sind fluorierte Verbindungen in der US-A 3 547 861 beschrieben, die fluorierte Acrylate und Polyacrylate betrifft, worin der fluorierte Rest sich von einem fluorierten Alkohol mit einer endständigen fluorierten Alkoxygruppe herleitet. Ähnliche Produkte für solche Verwendungen sind ebenfalls bekannt, worin der fluorierte Rest des Polyacrylats ein geradkettiger fluorierter Alkohol ist.

Die erfindungsgemäßen Verbindungen entsprechen der allgemeinen Formel (1)

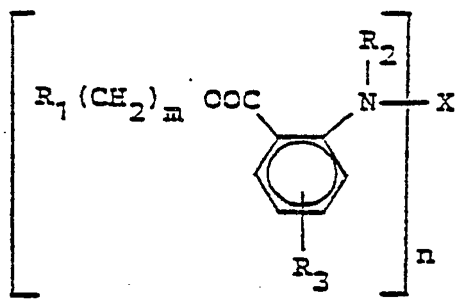

(1)

in welcher

$R_1$ Perfluoralkyl oder Perfluoralkoxy-perfluoralkyl mit jeweils insgesamt 2-20, vorzugsweise 4-14 C-Atomen,

$R_2$ Wasserstoff, geradkettiges oder verzweigtes $C_1$-$C_4$-Alkyl oder Phenyl,

$R_3$ Wasserstoff, oder einen oder mehrere nicht-farbgebende Reste aus der Gruppe Fluor, Chlor, Brom, niederes Alkyl, niederes Alkoxy, Acylamino, Carboxy- oder funktionell abgewandelte Carboxygruppen, Sulfo- oder funktionell abgewandelte Sulfogruppen oder Phenyl,

X den Rest einer aliphatischen oder aromatischen Mono- oder Polycarbonsäure, den Rest einer halogenalktiven, heterocyclischen Verbindung, wobei die nicht durch den Perfluoralkyl-anthranilsäureester-Rest substituierten Halogene in der heterocyclischen Verbindung durch die Reste von aliphatischen oder aromatischen Aminen substituiert sein können, oder einen Alkyl- oder Arylaminocarbonyl-Rest,

m eine ganze Zahl von 1 bis 6, vorzugsweise 1 bis 4 und

n eine ganze Zahl von 1 bis 3

bedeuten.

Bevorzugt sind die Perfluoralkylverbindungen der Formel (1), in welcher

$R_1$ eine Gruppe der Formel $C_lF_{2l+1}$—, eine Gruppe der Formel $H(C_2F_4)_o$— oder eine Gruppe der Formel $(CF_3)_2CFO(CF_2)_p$—,

l die Zahlen 6, 8, 10, 12, 14,

o die Zahlen 1, 2, 3 und 4 und

p ganze Zahlen von 2 bis 8,

$R_2$ Wasserstoff, Methyl, Ethyl oder Phenyl,

$R_3$ Wasserstoff oder einen oder mehrere Reste aus der Gruppe Chlor, Methyl, Methoxy, —COOH, —$COOCH_3$, —$SO_3H$, —$SO_2CC_6H_5$, —$SO_2NH_2$, CN oder Phenyl,

X für den Fall n = 1 eine Gruppe der Formeln —CO—$(NH)_a$—Y oder

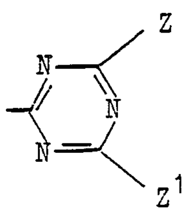

für den Fall n = 2 eine Gruppe der Formeln —CO—, —CO—$(NH)_a$—Y'—$(NH)_a$—CO— oder

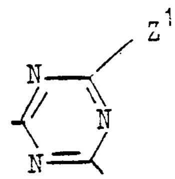

und für den Fall n = 3 eine Gruppe der Formel

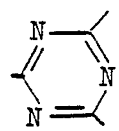

a 0 oder 1, Y Alkyl, Alkoxy, Phenyl, Chlorphenyl, Alkylphenyl, Y' Alkylen, Phenylen, Chlorphenylen, Alkylphenylen, Bis(4-cyclohex-1-yl) methan oder für den Fall a = 0 eine direkte Bindung, Z Chlor, Hydroxyethylamino, Bis-hydroxyethylamino, Z[1] Chlor, Hydroxyethylamino, Bis-hydroxyethylamino, Dialkylaminoalkylamino, Trialkylammoniumalkylamino, Sulfoethylamino, N-Alkyl-N-sulfoethylamino, Piperazinyl, N-Hydroxyethylpiperazinyl, N-Methyl-N-hydroxyethylpiperazinyl, Phenylamino, Sulfophenylamino, Di-sulfophenylamino, Alkylphenylamino, Sulfatoethylsulfonylphenylamino bedeuten.

Der Begriff « Perfluoralkyl » bzw. « Perfluoralkoxy » umfaßt sowohl solche Gruppen mit endständigen $-CF_3$ als auch mit endständigen $-CF_2H$ Gruppen. Die Begriffe « funktionell abgewandelte Carboxygruppen » und « funktionell abgewandelte Sulfogruppen » umfassen Cyano-, Carbonsäureester-, Carbonamid-, Mono- und Di-alkylcarbonamidgruppen bzw. Sulfonsäureester-, Sulfonamid-, Mono- und Di-alkylsulfonamidgruppen. Alkyl- und Alkoxygruppen enthalten, soweit nicht anders definiert, 1 bis 4 C-Atome. Unter einer Acylgruppe ist im wesentlichen eine $C_1$-$C_4$ Alkanoylgruppe zu verstehen. Die Carboxy- und Sulfogruppen können auch in Form der Salze mit farblosen Kationen vorliegen, beispielsweise als Ammonium-, Kalium- oder Natriumsalze.

Die erfindungsgemäßen Perfluoralkylanthranilsäureester sind schmutzabweisende Verbindungen, die gegenüber Wasser und Öl ein hohes Abweisungsvermögen besitzen und die auch nach wiederholten Wäschen und Trockenreinigungen auf der Faser verbleiben. Ein weiterer Vorteil der erfindungsgemäßen Verbindungen besteht darin, daß sie in Lösung oder als Dispersion auf die Fasermaterialien aufgebracht werden können oder sich durch Vermischen mit Pellets des thermoplastischen Kunststoffs und anschließendes Verformen zu Fasern oder Fäden einarbeiten lassen. Ein spezieller Vorteil der neuen schmutzabstoßenden Mittel besteht auch darin, daß sie ein einwandfreies Anfärben der Fasern oder Fäden, worin diese Mittel eingearbeitet werden, gestatten. Die neuen schmutzabstoßenden Mittel können auch mit zufriedenstellenden Ergebnissen zusammen mit einem Farbstoff aus einem Bade aufgebracht werden.

Die Herstellung der erfindungsgemäßen Verbindungen (1) erfolgt durch Umsetzung von Isatosäureanhydriden (2) mit Perfluoralkyl- oder Perfluoralkoxy-perfluoralkyl-alkoholen zu den Perfluoralkyl-anthranilsäureester-Zwischenprodukten (3), deren Acylierung mit Säurehalogeniden aliphatischer oder aromatischer Mono- oder Polycarbonsäuren oder deren Kondensation mit einer halogenaktiven, heterocyclischen Verbindung, wie zum Beispiel Cyanurchlorid, oder deren Umsetzung mit aliphatischen oder aromatischen Mono- oder Diisocyanaten, wie im folgenden Reaktionsschema an Hand der Umsetzung mit Cyanurchlorid beispielhaft dargestellt.

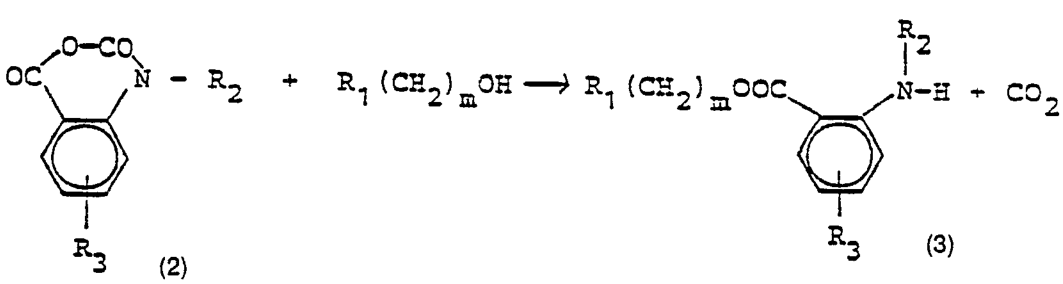

(2) (3)

(3) 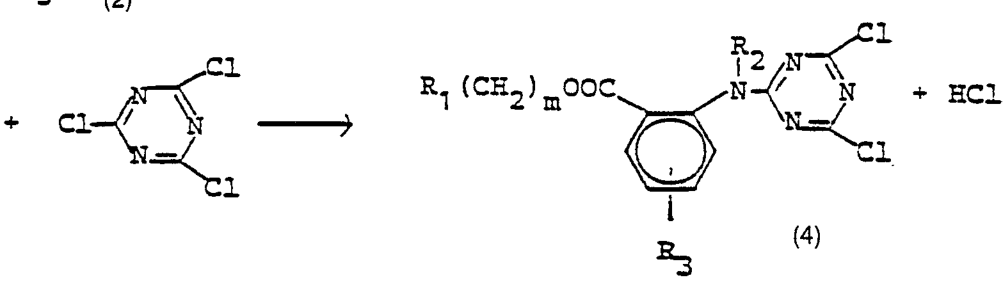

(4)

In den vorstehenden Formeln (2), (3) und (4) haben $R_1$, $R_2$, $R_3$ und m die eingangs angegebene Bedeutung.

Die als bevorzugt aufgeführten Verbindungen werden entsprechend hergestellt durch Umsetzung von 1, 2 oder 3 Mol der Verbindungen der Formel (3) mit Verbindungen der folgenden Formeln $CO(Hal)_2$ ; Hal—CO—Y ; Hal—CO—Y'—CO—Hal ; OCN—Y ; OCN—Y'—NCO oder Cyanurchlorid. Hal bedeutet hierbei Chlor oder Brom und die übrigen Symbole haben die zuvor angegebenen Bedeutungen. Bei den Verbindungen der Formel (4) die durch Umsetzung von Verbindungen der Formel (3) mit Cyanurchlorid erhalten werden, lassen sich die beiden Chloratome jedes für sich nach bekannten Verfahren gegen den Rest des Anthranilsäureesters der Formel (3) oder gegen den Rest eines Amins entsprechend der Bedeutung von Z und $Z^1$ austauschen.

Zur Herstellung der Zwischenprodukte (3) werden die in der Literatur mehrfach beschriebenen Isatosäureanhydride (2) (ERDMANN, B. 32, 2164, DE-C 110 577 ; Frdl. 5, 148) mit einer äquimolekularen Menge eines Perfluoralkyl- oder Perfluoralkoxy-perfluoralkyl-alkohols bei Temperaturen bis 200 °C bis zur Beendigung der $CO_2$-Entwicklung oder vorteilhafter und schonender in Gegenwart alkalischer Katalysatoren, wie z. B. Natriummethylat, bei Temperaturen bis 100 °C umgesetzt.

Die Acylierung der Zwischenprodukte (3) mit den Chloriden aliphatischer oder aromatischer Mono- oder Dicarbonsäuren erfolgt in organischen Lösungsmitteln, vorteilhaft in Toluol oder Xylol, in Gegenwart von Protonenfängern, zum Beispiel Pyridin, bei Temperaturen von 50-60 °C.

Die Umsetzung der Zwischenprodukte (3) mit Cyanurchlorid wird vorteilhaft in Essigsäureethylester ohne Protonenacceptoren bei 5-10 °C durchgeführt. Zum weiteren Austausch der Chloratome in Verbindung (4) gegen die Reste aliphatischer oder aromatischer Amine wendet man vorteilhaft höher siedende Lösungsmittel wie Toluol, Chlorbenzol oder o-Dichlorbenzol an. Protonenfänger, wie Pyridin oder Triethylamin, wirken sich eher nachteilig auf das Gelingen dieser Reaktionen aus.

Die Kondensation der Zwischenprodukte (3) mit aliphatischen oder aromatischen Mono- oder Diisocyanaten erfolgt vorteilhaft in polaren aprotischen Lösungsmitteln, wie Dimethylformamid oder N-Methyl-pyrrolidon, bei Temperaturen von 50-60°.

Verbindungen, die der vorliegenden Erfindung entsprechen, haben beispielsweise die Formeln :

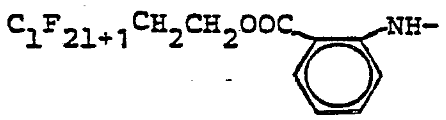 = A    $l = 6, 8, 10, 12$

$A-COC_3H_7$, 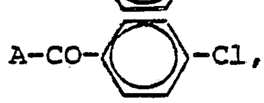, $A-COOCH_3$, $A-CO(CH_2)_3CO-A$ ,

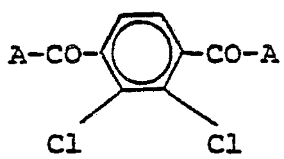 , 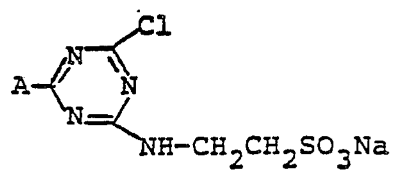

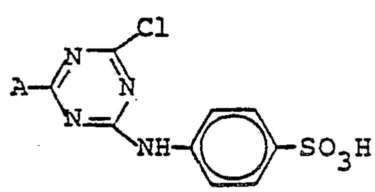 , 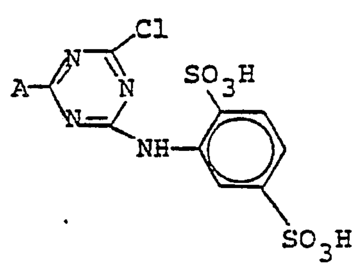

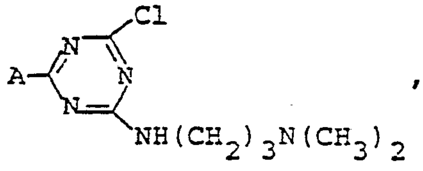 , 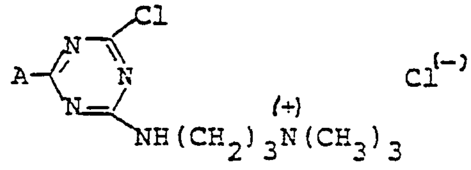 ,

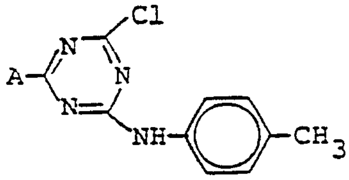 , 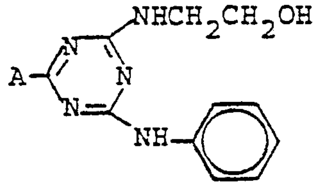

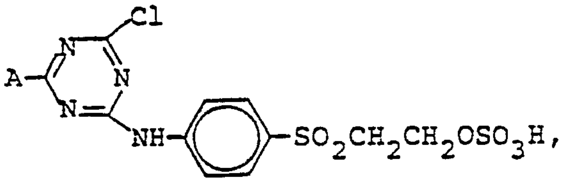 , 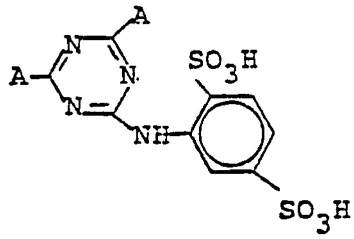

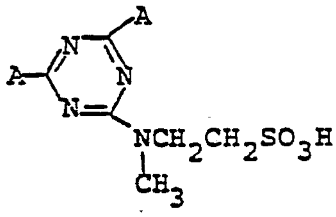 , 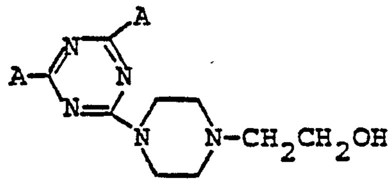

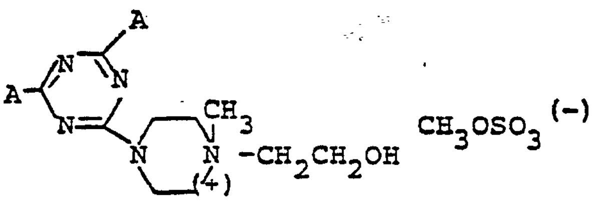 , 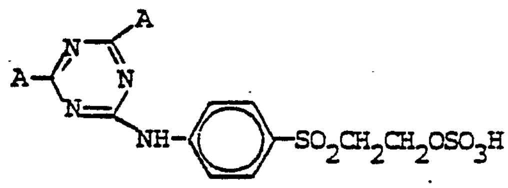

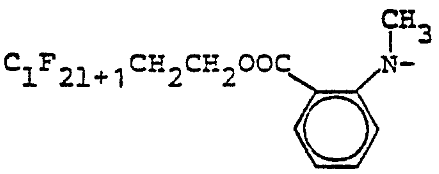 = B l = 6, 8, 10, 12

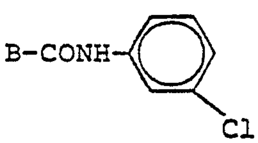 , 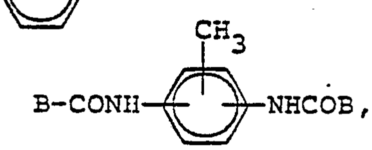 , 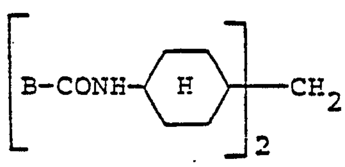

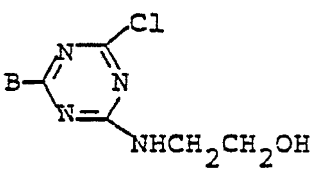 , 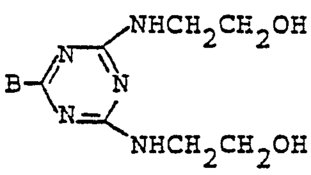

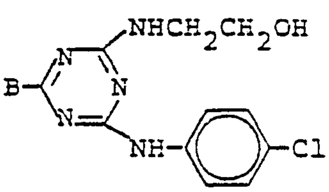 , 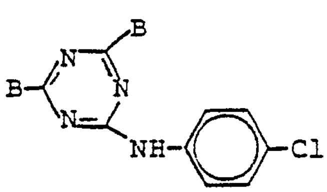

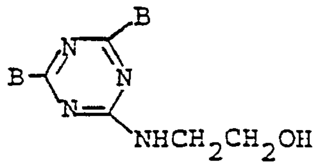

,

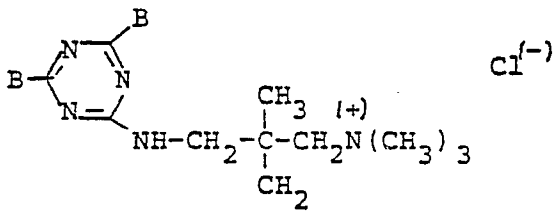

.

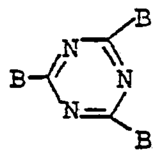

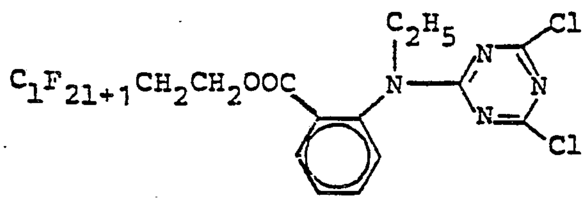

, l = 6, 8, 10, 12

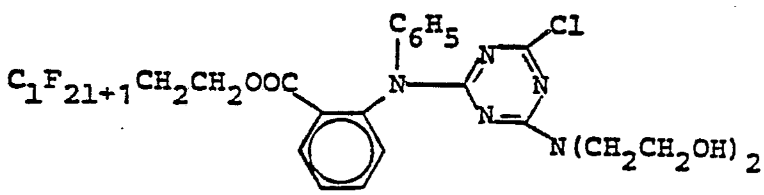

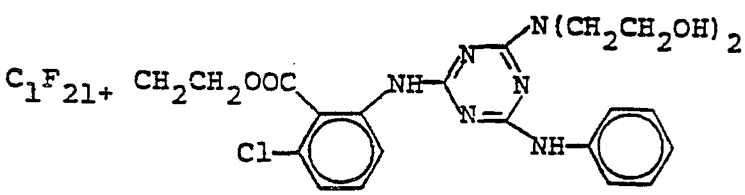

Die erfindungsgemäßen Verbindungen der Formel (1) eignen sich zur gleichzeitigen Hydrophobierung und Oleophobierung von synthetischen und natürlichen Fasern und Geweben, insbesondere für Polyester, Polyamid, Polyacrylnitril und Wolle. Die Applikation dieser Verbindungen auf das Textilmaterial erfolgt nach bekannten Verfahren durch Imprägnieren mit einer Lösung der Verbindungen der Formel (1) in Wasser oder einem geeigneten organischen Lösungsmittel, vorzugsweise in Aceton oder Dimethylformamid. Man kann die Verbindungen der Formel (1) aber auch in Form wäßriger Dispersionen einsetzen. Das Textilmaterial wird nach dem Imprägnieren abgequetscht, getrocknet und thermofixiert. Besonders bevorzugt ist die gleichzeitige Mitverwendung der Verbindungen der Formel (1) in einem üblichen Faserpräparationsmittel. Die Auflage der Verbindungen der Formel (1) auf dem Textilmaterial beträgt im allgemeinen 0,05 bis 1 Gew.-%, berechnet auf Gehalt an Fluor in den Verbindungen der Formel (1).

Es wird angenommen, daß die schmutzabstoßenden Eigenschaften den Thermoplasten durch die vorliegenden Verbindungen der Formel (1) auf Grund ihrer Funktion verliehen werden, die Oberflächenenergie der Thermoplasten zu vermindern. Dieser Effekt kann durch ein Tempern bei Temperaturen oberhalb der Glasübergangstemperatur der Thermoplasten und unterhalb der Zersetzungstemperatur sowohl der Thermoplasten als auch des schmutzabstoßenden Mittels verbessert werden.

Geeignete Zeiten für ein solches Tempern liegen im Bereich von etwa 1 bis 240 Minuten. Die Temperaturen des Temperns liegen typischerweise bei etwa 100 bis 220 °C.

Eine weitere Verbesserung des Effektes der erfindungsgemäßen schmutzabstoßenden Mittel, die eine Hydroxylgruppe enthalten, bekommt man bei Mitverwendung eines difunktionellen oder trifunktionellen Epoxids oder Isocyanats in dem flüssigen Medium, das das schmutzabstoßende Mittel enthält und in welches die Faser oder der andere thermoplastische Gegenstand eingetaucht wird oder mit der diese besprüht oder anderweitig behandelt werden, und zwar zusammen mit einem Katalysator, wie einem Amin, um die Reaktion der Hydroxylgruppe mit einer Epoxydgruppe oder Isocyanatgruppe beim anschließenden Tempern zu fördern.

Herstellungsbeispiele

Tabelle 1

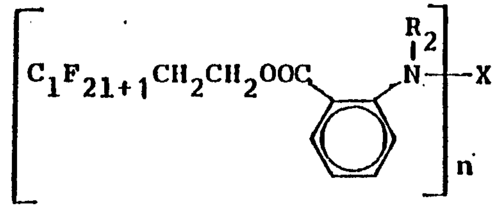

l = 6, 8, 10, 12

| Lfd.Nr. | $R_2$ | X | n | Methode | Ausbeute % d.Th. | Analyse % F; % Cl; | % N |
|---|---|---|---|---|---|---|---|
| (10)*) | H | H | 1 | A | 96,8 | Ber. 54,7; | 2,5 |
| | | | | | | Gef. 53,9; | 2,6 |
| (11)*) | $CH_3$ | H | 1 | A' | 85,3 | Ber. 53,6; | 2,4 |
| | | | | | | Gef. 52,8; | 2,6 |
| (12) | $CH_3$ | $CH_3CO-$ | 1 | B | 93,1 | Ber. 49,8; | |
| | | | | | | Gef. 48,9; | |
| (13) | $CH_3$ | $C_6H_5CO-$ | 1 | B' | 96,4 | Ber. 45,3; | |
| | | | | | | Gef. 44,6; | |
| (14) | $CH_3$ | $C_2H_5OCO-$ | 1 | B' | 89,3 | Ber. 47,5; | |
| | | | | | | Gef. 48,2; | |
| (15) | $CH_3$ | -CO- | 2 | C | 89,0 | Ber. 52,2; | |
| | | | | | | Gef. 51,4; | |
| (16) | H | -COCO- | 2 | C | 84,4 | Ber. 52,4; | |
| | | | | | | Gef. 51,9; | |
| (17) | $CH_3$ | -"- | 2 | C | 81,7 | Ber. 51,0; | |
| | | | | | | Gef. 51,0; | |
| (18) | H | $-CO(CH_2)_2CO-$ | 2 | C | 97,8 | Ber. 51,2; | |
| | | | | | | Gef. 50,7; | |

*) Herstellung der Zwischenprodukte der allgemeinen Formel (3)

Tabelle 1 (Fortsetzung)

| Lfd.Nr. | $R_2$ | X | n | Methode | Ausbeute % d.Th. | Analyse % F; % Cl; % N |
|---|---|---|---|---|---|---|
| (19) | $CH_3$ | $-CO(CH_2)_2CO-$ | 2 | C | 96,7 | Ber. 50,1<br>Gef. 49,2 |
| (20) | H | $-CO(CH_2)_4CO-$ | 2 | C | 96,3 | Ber. 50,1<br>Gef. 49,8 |
| (21) | $CH_3$ | -"- | 2 | C | 97,3 | Ber. 48,8<br>Gef. 47,9 |
| (22) | H | 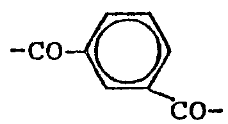 | 2 | C | 96,8 | Ber. 49,3<br>Gef. 48,8 |
| (23) | $CH_3$ | -"- | 2 | C | 97,6 | Ber. 48,0<br>Gef. 47,5 |
| (24) | $CH_3$ | 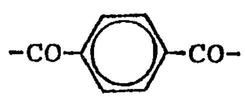 | 2 | C | 97,3 | Ber. 48,0<br>Gef. 47,6 |
| (25) | H | 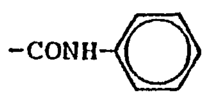 | 1 | D | 98,7 | Ber. 45,4<br>Gef. 44,4 |
| (26) | H | 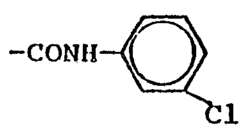 | 1 | D | 100 | Ber. 43,3<br>Gef. 43,5 |

Tabelle 1 (Fortsetzung)

| Lfd.Nr. | $R_2$ | X | | n | Methode | Ausbeute % d.Th. | Analyse % F; % Cl; % N |
|---|---|---|---|---|---|---|---|
| (27) | H | $-CONH(CH_2)_6NHCO-$ | | 2 | D' | 100 | Ber. 48,2<br>Gef. 47,8 |
| (28) | H | 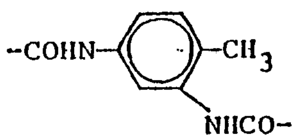 | 80% 2,4-<br>20% 2,6- | 2 | D' | 97,7 | Ber. 47,6<br>Gef. 47,0 |
| (29) | H | 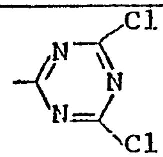 | | 1 | E | 98,7 | Ber. 43,6; 9,85; 7,78<br>Gef. 43,4; 9,8; 7,9 |
| (30) | $CH_3$ | -"- | | 1 | E | 99,6 | Ber. 42,6; 9,73; 7,69<br>Gef. 42,2; 9,5; 7,5 |
| (31) | H | 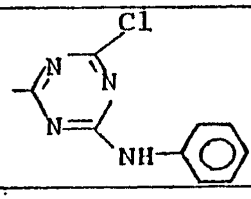 | | 1 | F | 100 | Ber. 40,43<br>Gef. 40,2 |
| (32) | $CH_3$ | -"- | | 1 | F | 92,8 | Ber. 39,7<br>Gef. 38,9 |
| (33) | H | 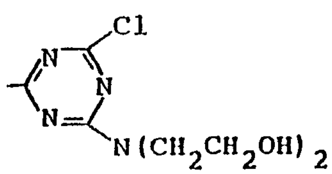 | | 1 | F | 90,8 | Ber. 39,8<br>Gef. 39,4 |

Tabelle 1 (Fortsetzung)

| Lfd.Nr. | $R_2$ | X | n | Methode | Ausbeute % d.Th. | Analyse % F; % Cl; % N |
|---|---|---|---|---|---|---|
| (34) | $CH_3$ | 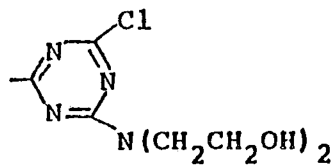 | 1 | F | 95,2 | Ber. 39,1<br>Gef. 38,9 |
| (35) | H | 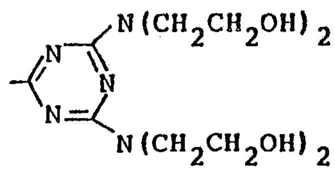 | 1 | G | 64,7 | Ber. 36,6<br>Gef. 36,5 |
| (36) | $CH_3$ | -"- | 1 | G | 89,4 | Ber. 36,0<br>Gef. 36,3 |
| (37) | H | 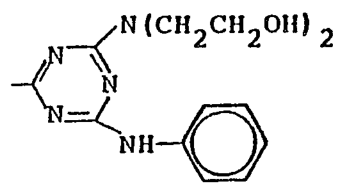 | 1 | G | 70,2 | Ber. 37,1<br>Gef. 36,4 |
| (38) | $CH_3$ | -"- | 1 | G | 67,5 | Ber. 36,5<br>Gef. 35,9 |
| (39) | H | 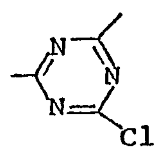 | 2 | H | 97,6 | Ber. 50,0<br>Gef. 49,8 |

Tabelle 1 (Fortsetzung)

| Lfd.Nr. | $R_2$ | X | n | Methode | Ausbeute % d.Th. | Analyse % F; % Cl; % N |
|---|---|---|---|---|---|---|
| (40) | $CH_3$ | 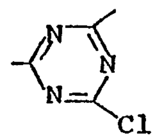 | 2 | H | 100 | Ber. 48,9; 5,46<br>Gef. 48,1; 5,5 |
| (41) | H | 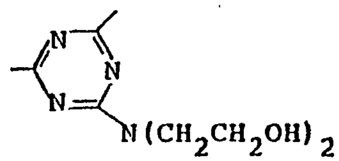 | 2 | I | 78,3 | Ber. 47,4<br>Gef. 47,1 |
| (42) | $CH_3$ | -"- | 2 | I | 97,2 | Ber. 46,5<br>Gef. 46,4 |
| (43) | H | 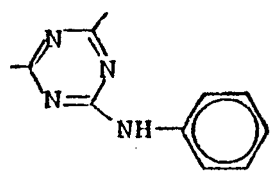 | 2 | I | 80,5 | Ber. 49,9<br>Gef. 50,0 |
| (44) | $CH_3$ | -"- | 2 | I | 91,7 | Ber. 46,9<br>Gef. 46,3 |
| (45) | H | 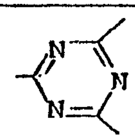 | 3 | J | 90,3 | Ber. 52,6<br>Gef. 52,0 |

Methode A

Zu einer Mischung aus 1 Mol des Perfluoralkylethanols $C_lF_{2l+1}CH_2CH_2OH$, l = 6, 8, 10, 12 und 1 Mol Isatosäureanhydrid werden 0,1 Mol Natriummethylat zugegeben. Die Reaktionsmischung wird unter Rührung auf 100° angeheizt wobei eine lebhafte Gasentwicklung einsetzt. Man rührt 1 Stunde bei 100° nach, läßt auf Raumtemperatur erkalten und versetzt mit 1 l Toluol und 50 ml Wasser. Unter Rührung wird mit 6 ml Eisessig neutralisiert und die wäßrige Phase bei 40-50° abgetrennt. Die organische Phase wird noch dreimal mit je 500 ml Wasser in der Wärme ausgerührt, über Natriumsulfat getrocknet und im Vakuum eingedampft. Ausbeute : 548 g der Verbindung Nr. (10) in Form einer bräunlichen halbfesten Masse. Bei Destillation im Vakuum geht ein Hauptlauf bei 130-170°/0,4-1,0 Torr über, der zu einer farblosen Masse erstarrt.

Methode A'

Unter Verwendung von N-Methylisatosäureanhydrid wird analog Methode A gearbeitet. Nach dem Erkalten auf Raumtemperatur gibt man 7 ml Eisessig zu und destilliert anschließend im Vakuum. Bei 130-170°/0,4-0,6 Torr gehen 489 g einer gelblichen, bei Raumtemperatur glasartig erstarrenden Masse über (Verbindung Nr. 11).

Methode B

29 g des N-Methyl-perfluoralkylanthranilsäureesters (11) werden in 150 ml Toluol gelöst und mit 9,4 ml Acetanhydrid und 4,8 ml Pyridin versetzt. Man kocht 5 Stunden unter Rückfluß, dampft im Rotavapor das Lösungsmittel ab und rührt das verbleibende gelbliche Öl bei 50° mehrfach mit Wasser aus. Nach dem Trocknen bei 60° im Vakuum werden 29,0 g eines gelben Öls erhalten (Verbindung Nr. 12).

Methode B'

Analog Methode B wird mit 7,03 g Benzoylchlorid gearbeitet und das Reaktionsgemisch 6 Stunden bei 55-60° nachgerührt. Es werden 33,0 g eines gelben Öls erhalten (Verbindung Nr. 13).

Methode C

0,1 Mol des Anthranilsäureesters werden in 350 ml Toluol gelöst bzw. suspendiert und unter Rührung mit 0,05 Mol des Dichlorids und 0,12 Mol Pyridin versetzt. Man erhitzt 6-7 Stunden auf 55-60°, läßt erkalten und saugt das ausgefallene Reaktionsprodukt ab. Es wird mit Toluol und Wasser gewaschen und im Vakuum bei 60° getrocknet. Wenn sich das Reaktionsprodukt nicht absaugen läßt, wird das Lösungsmittel im Vakuum abdestilliert und der verbleibende zumeist harzige Rückstand evtl. unter Erwärmung und unter Zuhilfenahme eines elektrisch betriebenen Kneters gründlich mit Wasser bis zur neutralen Reaktion ausgewaschen. Das letzte Waschwasser wird auf Abwesenheit von $Cl^-$-Ionen überprüft.

Methode D

0,1 Mol des Anthranilsäureesters werden in 20 ml Dimethylformamid tropfenweise mit 0,11 Mol des Monoisocyanats versetzt. Man erhitzt 3 Stunden auf 60-80 °C, verdampft anschließend das Lösungsmittel im Vakuum und verrührt den Rückstand, gegebenenfalls nach mechanischer Zerkleinerung, mehrere Stunden mit Wasser. Nach dem Absaugen wird mit Wasser gewaschen und im Vakuum bei 60° getrocknet.

Methode D'

0,1 Mol des Anthranilsäureesters werden in 30 ml Dimethylformamid gelöst und mit 0,1 Mol des Diisocyanats tropfenweise versetzt. Man erwärmt auf 60° und verrührt 3 Stunden bei dieser Temperatur. Nach dem Erkalten gießt man das Reaktionsgemisch auf 200 ml Wasser, verrührt mehrere Stunden, saugt ab, wäscht mit Wasser nach und trocknet bei 60° im Vakuum.

Methode E

0,1 Mol des Anthranilsäureesters werden in 350 ml Essigsäureethylester gelöst, auf 5-10° abgekühlt und mit 0,1 Mol Cyanurchlorid versetzt. Das Reaktionsgemisch wird 1 Stunde bei 5-10° und weitere 24 Stunden bei Raumtemperatur verrührt. Anschließend wird im Rotavapor bis zum konstanten Gewicht bei 50° eingedampft. Die Reaktionsprodukte sind farblose bis schwach bräunlich gefärbte Pulver.

Methode F

0,1 Mol des N-(Dichlor-triazinyl)-anthranilsäureesters (29) bzw. (30) werden in 350 ml Toluol bei 60-70° gelöst, mit 0,2 Mol eines aliphatischen oder aromatischen Amins versetzt und 4-8 Stunden bei 95-100° verrührt. Das Reaktionsgemisch wird im Vakuum eingedampft und der Rückstand zweimal mit 350 1n Salzsäure durchgerührt. Das abgesaugte Reaktionsprodukt wird mit Wasser frei von $Cl^-$-Ionen gewaschen und bei 60° im Vakuum getrocknet.

Methode G

0,1 Mol des N-(Dichlortriazinyl)-anthranilsäureesters (29) bzw. (30) werden in 350 ml Toluol bei 60-70° gelöst, mit 0,2 Mol eines aliphatischen oder aromatischen Amins versetzt und 4-8 Stunden bei 95-100° verrührt. Anschließend gibt man 0,2-0,3 Mol des gleichen oder eines anderen aliphatischen Amins zu und erhitzt das Reaktionsgemisch ca. 65 Stunden auf 100°. Das Toluol wird im Vakuum abdestilliert, der Rückstand gegebenenfalls in der Wärme mehrmals mit 350 ml 1n Salzsäure durchgerührt, abgesaugt, mit Wasser $Cl^-$-frei gewaschen und im Vakuum bei 60° getrocknet.

Methode H

0,1 Mol des N-(Dichlortriazinyl)-anthranilsäureesters (29) bzw. (30) werden in 200 ml Toluol suspendiert, mit 0,1 Mol des entsprechenden Anthranilsäureesters versetzt und unter Durchleiten von Stickstoff 12 Stunden unter Rückflußsieden gerührt. Das Toluol wird im Vakuum abdestilliert und der Rückstand bei 50-60° im Vakuum getrocknet.

Methode I

Zu 0,1 Mol der Chlortriazinylverbindung (39) bzw. (40) in 200 ml Toluol werden 0,2 Mol eines aliphatischen oder aromatischen Amins zugesetzt und das Reaktionsgemisch 24 Stunden bei Siedetemperatur verrührt. Das Toluol wird im Vakuum abdestilliert und der Rückstand, gegebenenfalls in der Wärme mehrmals mit 350 ml 1n Salzsäure durchgerührt. Man saugt ab, wäscht mit Wasser frei von $Cl^-$-Ionen und trocknet im Vakuum bei 50-60°.

Methode J

0,1 Mol der Chlortriazinylverbindung (39) in 200 ml o-Dichlorbenzol suspendiert werden, mit 0,1 Mol des Anthranilsäureesters (10) versetzt und unter Durchleiten von Stickstoff insgesamt 36 Stunden unter Rückflußsieden verrührt. Das Lösungsmittel wird im Vakuum abdestilliert und der Rückstand im Vakuum bei 80° bis zum konstanten Gewicht getrocknet.

## Anwendungsbeispiele

Auf Gewebe aus Polyamid-6-Filamenten und Polyester werden mit einem Foulard die in folgender Tabelle 2 aufgeführten Verbindungen aus acetonischer oder isopropanolischer Lösung mit einer Flottenaufnahme von 40-50 % aufgebracht. Die Produktmenge wird so gewählt, daß sich nach dem Trocknen ca. 0,06 % Fluor auf den Geweben befinden. In den acetonischen oder isopropanolischen Lösungen sind ca. 0,6 g Substanz in 250 ml enthalten.

Die Gewebe werden luftgetrocknet, 1 Minute bei 160 °C kondensiert und anschließend einer 3stündigen Wasserbehandlung bei 100° in einer Waschmaschine unterworfen (KW). Die nach den einzelnen Ausrüstungsschritten gefundenen Ölabweisungswerte nach AATCC (Testmethode Nr. 118-1966 der American Association of Textile Colorists and Chemists) sind in der folgenden Tabelle 2 aufgeführt. Dabei bedeutet 3 ungenügende, 4 zufriedenstellende, 5 gute und 6 sehr gute Ölabweisung, 7 steht für Spitzenwerte.

Tabelle 2

| Lfd. Nr. nach Tabelle 1 | Polyamid | | | Polyester | | |
|---|---|---|---|---|---|---|
| | luftgetrocknet | 1 Min. 160° | 3 Stdn. KW | luftgetrocknet | 1 Min. 160° | 3 Stdn. KW |
| (17) | 6 - 7 | 6 | 6 - 7 | 6 | 6 | 6 |
| (19) | 5 | 5 | 4 - 5 | 4 - 5 | 5 | 5 |
| (21) | 5 | 5 | 6 | 4 - 5 | 5 | 5 |
| (23) | 6 | 6 | 6 | 5 | 5 | 6 |
| (33) | 5 | 5 - 6 | 5 | | | |

(Fortsetzung)

| Lfd. Nr. nach Tabelle 1 | Polyamid luftgetrocknet | 1 Min. 160° | 3 Stdn. KW | Polyester luftgetrocknet | 1 Min. 160° | 3 Stdn. KW |
|---|---|---|---|---|---|---|
| (34) | 4 - 5 | 4 | 5 | | | |
| (40) | 4 - 5 | 4 | 5 | | | |
| (42) | 5 - 6 | 5 | 5 - 6 | 5 | 5 | 5 |

Die folgenden Beispiele zeigen, daß die erfindungsgemäßen Verbindungen auch in Form einer Dispersion appliziert werden können und daß dieser Schritt mit einem Färbeverfahren kombiniert werden kann.

## Beispiel 1

PA-Flocke, zu färben im Packsystem, Flottenverhältnis 1 : 10-1 : 15,

1 % Säurefarbstoff
1-2 ml/l Ammoniak 25 %ig, (pH 8-8,5)
3-5 ml/l $CH_3COOH$ 60 %ig, pH 4,5-5,0
evtl. 0,5-1 g/l eines Egalisiermittels auf Basis Stearylaminethoxilat, EO 18-22,
2-5 g/l einer Emulsion auf Basis Benzoxipropionitril,
0,1-0,2 % der erfindungsgemäßen Verbindung (bezogen auf Warengewicht).

Die Färbung wird bei 30 °C begonnen mit der Zugabe von Ammoniak, Egalisiermittel und Emulsion. Man läßt die Flotte ca. 5 Min. durchmischen, gibt den gelösten Farbstoff in das Färbebad und heizt mit ca. 1°/Min. bis auf 80 °C Badtemperatur auf. Durch Zugabe von 3-5 ml/l $CH_3COOH$ 60 %ig wird der pH auf 5-6 eingestellt, die Dispersion der erfindungsgemäßen Verbindung zugefügt und nach 5 Minuten Mischzeit weiter auf eine Färbetemperatur von 90°-120 °C, vorzugsweise auf 98°-110° aufgeheizt. Nach ca. 60 Minuten wird die Färbung gespült und getrocknet. Ein Trocknen bei Temperaturen von 120°-160° während 3-5 Minuten ist vorzuziehen.

## Beispiel 2

PA-Flocke-Packsystem, Flottenverhältnis 1 : 10-1 : 15

1 % Säurefarbstoff
1-2 ml/l Ammoniak 25 %ig (pH 8-8,5)
evtl. 0,5-1 g/l eines Egalisiermittels auf Basis Stearylaminethoxilate mit 18-22 EO
3-5 ml/l $CH_3COOH$ 60 %ig

Die Färbung wird bei 30 °C begonnen mit der Zugabe von Ammoniak, Egalisiermittel, Farbstoff und auf 98 °C aufgeheizt. Nach 60 Minuten wird die Färbung, die jetzt abgeschlossen ist, auf 80 °C abgekühlt und

3-5 ml/1 Essigsäure 60 %ig (pH 4,5-5,0)
2-5 g/l Emulsion auf Basis Phenyloxipropionitril oder Benzoxipropionitril,
0,1-0,2 % der erfindungsgemäßen Verbindung (berechnet auf Warengewicht)

dem Färbebad zugefügt. Nach einer Mischzeit von ca. 5 Minuten heizt man die Flotte wieder auf 90°-120 °C, vorzugsweise auf 98°-110 °C, auf und verweilt ca. 60 Minuten, kühlt ab auf ca. 60 °C, spült, trocknet bei Temperaturen von 120°-160 °C während 3-5 Minuten.

## Beispiel 3

Färbung PES-Garn, Packsystem, Flottenverhältnis 1 : 8-1 : 12

1 % Dispersionsfarbstoff
ca. 1 ml/l $CH_3COOH$ 60 %ig (ph 5.2)
2 g/l Carrier auf Basis Diphenol oder o-Phenylphenol
0,1-0,2 % der erfindungsgemäßen Verbindung (berechnet auf Warengewicht)

Man beginnt die Färbung vorzugsweise bei Temperaturen um 50°-60 °C, fügt Essigsäure und Farbstoff hinzu und heizt nach 5 Minuten Mischzeit auf 80°-85 °C die Flotte auf. Es werden der Carrier emulgiert der Färbeflotte und die Dispersion der erfindungsgemäßen Verbindung zugefügt, ca. 5 Minuten vermischt und auf 130 °C aufgeheizt. Nach 60 Minuten wird die Färbung beendet und auf 80 °C die Flotte abgekühlt, das Material gespült und bei 120°-160 °C während 3-5 Minuten getrocknet.

In allen drei Beispielen wurden nach der Methode 3M/AATCC folgende Werte für die Oleophobierung gemessen :

Verbindung Nr. (33) : 100/5

Verbindung Nr. (39) : 120/6

**Patentansprüche**

1. Perfluoralkyl-anthranilsäureester der Formel (1)

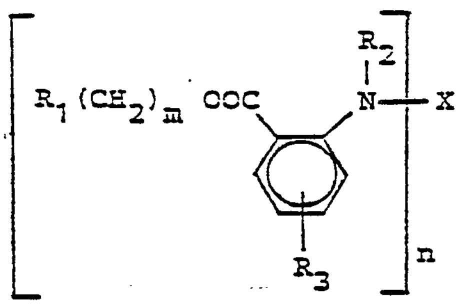

(1)

in welcher

$R_1$ Perfluoralkyl oder Perfluoralkoxy-perfluoralkyl mit jeweils insgesamt 2-20, vorzugsweise 4-14 C-Atomen,

$R_2$ Wasserstoff, geradkettiges oder verzweigtes $C_1$-$C_4$-Alkyl, oder Phenyl

$R_3$ Wasserstoff, oder einen oder mehrere nicht-farbgebende Reste aus der Gruppe Fluor, Chlor, Brom, niederes Alkyl, niederes Alkoxy, Acylamino, Carboxy- oder funktionell abgewandelte Carboxygruppen, Sulfo- oder funktionell abgewandelte Sulfogruppen oder Phenyl

X den Rest einer aliphatischen oder aromatischen Mono- oder Polycarbonsäure, den Rest einer halogenaktiven, heterocyclischen Verbindung, wobei die nicht durch den Perfluoralkyl-anthranilsäureester-Rest substituierten Halogene in der heterocyclischen Verbindung durch die Reste von aliphatischen oder aromatischen Aminen substituiert sein können, oder einen Alkyl- oder Arylaminocarbonyl-Rest,

m eine ganze Zahl von 1 bis 6, vorzugsweise 1 bis 4, und

n eine ganze Zahl von 1 bis 3

bedeuten.

2. Perfluoralkyl-anthranilsäureester der Formel (1) nach Anspruch 1, in welcher

$R_1$ eine Gruppe der Formel $C_lF_{2l+1}$—, eine Gruppe der Formel $H(C_2F_4)_o$— oder eine Gruppe der Formel $(CF_3)_2CFO(CF_2)_p$—,

l die Zahlen 6, 8, 10, 12, 14

o die Zahlen 1, 2, 3 und 4 und

p ganze Zahlen von 2 bis 8,

$R_2$ Wasserstoff, Methyl, Ethyl oder Phenyl,

$R_3$ Wasserstoff oder einen oder mehrere Reste aus der Gruppe Chlor, Methyl, Methoxy, —COOH, —$COOCH_3$—$SO_3H$, $SO_2OC_6H_5$, $SO_2NH_2$, CN oder Phenyl,

X für den Fall n = 1 eine Gruppe der Formeln —CO—$(NH)_a$—Y oder

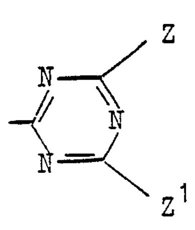

für den Fall n = 2 eine Gruppe der Formeln —CO— ; —CO—$(NH)_a$—Y'—$(NH)_a$—CO— oder

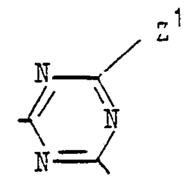

und für den Fall n = 3 eine Gruppe der Formel

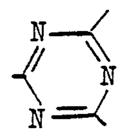

a 0 oder 1, Y Alkyl, Alkoxy, Phenyl, Chlorphenyl, Alkylphenyl, Y' Alkylen, Phenylen, Chlorphenylen, Alkylphenylen, Bis(4-cyclohex-1-yl) methan oder für den Fall a = 0 eine direkte Bindung, Z Chlor, Hydroxyethylamino, Bis-hydroxyethylamino, $Z^1$ Chlor, Hydroxyethylamino, Bis-hydroxyethylamino, Dialkylaminoalkylamino, Trialkylammoniumalkylamino, Sulfoethylamino, N-Alkyl-N-sulfoethylamino, Piperazinyl, N-Hydroxyethylpiperazinyl, N-Methyl-N-hydroxyethylpiperazinyl, Phenylamino, Sulfophenylamino, Disulfophenylamino, Alkylphenylamino, Sulfatoethylsulfonylphenylamino bedeuten.

3. Verfahren zur Herstellung der Perfluoralkyl-anthranilsäureester der Formel (1) nach Anspruch 1, dadurch gekennzeichnet, daß man Isatosäureanhydride (2) mit Perfluoralkyl- oder Perfluoralkoxy-perfluoralkyl-alkoholen bevorzugt in Gegenwart alkalischer Katalysatoren zu den Perfluoralkyl-anthranilsäureester-Zwischenprodukten (3) umsetzt

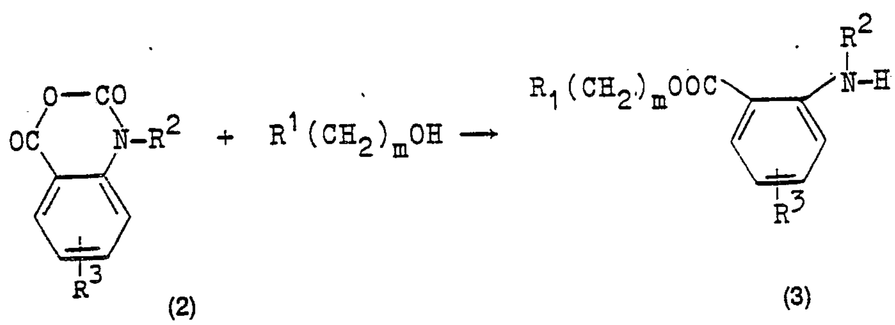

(2) (3)

und diese

a) mit Säurehalogeniden aliphatischer oder aromatischer Mono- oder Polycarbonsäuren acyliert oder

b) mit halogenaktiven, heterocyclischen Verbindungen bevorzugt Cyanurchlorid, kondensiert und in den Kondensationsprodukten die Halogenatome gegebenenfalls durch Reste von gegebenenfalls substituierten aliphatischen oder aromatischen Aminen austauscht oder

c) mit aliphatischen oder aromatischen Mono- oder Diisocyanaten weiter umsetzt.

4. Verwendung der Perfluoralkyl-anthranilsäureester der Formel (1) nach Anspruch 1 zur öl- und wasserabweisenden Ausrüstung von Textilmaterialien.

**Claims**

1. A perfluoroalkyl ester of an anthranilic acid, of the formula (1)

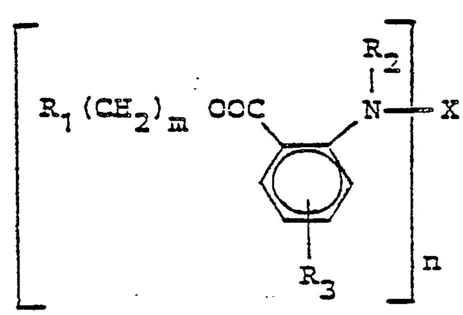

(1)

in which

$R_1$ is perfluoroalkyl or perfluoroalkoxy-perfluoroalkyl, in each case having a total of 2-20, preferably 4-14, carbon atoms,

$R_2$ is hydrogen, straight-chain or branched $C_1$-$C_4$-alkyl or phenyl,

$R_3$ is hydrogen or one or more non-chromophoric radicals from the group of fluorine, chlorine, bromine, lower alkyl, lower alkoxy, acylamino, carboxyl or functionally modified carboxyl groups, sulfo or functionally modified sulfo groups, or phenyl,

X is the radical of an alipatic or aromatic monocarboxylic or polycarboxylic acid, the radical of a halogen-active heterocyclic compound or an alkylaminocarbonyl or arylaminocarbonyl radical, the halogen atoms in the heterocyclic compound which are not substituted by the perfluoroalkyl ester of anthranilic acid may be substituted by aliphatic or aromatic amines,

m is an integer from 1 to 6, preferably 1 to 4 and

n is an integer from 1 to 3.

2. A perfluoroalkyl ester of an anthranilic acid of the formula (1) as claimed in claim 1, in which

$R_1$ is a group of the formula $C_lF_{2l+1}$—, a group of the formula $H(C_2F_4)_o$— or a group of the formula $(CF_3)_2CFO(CF_2)_p$—,

l is 6, 8, 10, 12 or 14,

o is 1, 2, 3 and 4 and

p is an integer from 2 to 8,

$R_2$ is hydrogen, methyl, ethyl or phenyl,

$R_3$ is hydrogen or one or more radicals from the group comprising chlorine, methyl, methoxy, —COOH, —$COOCH_3$, —$SO_3H$, —$SO_2OC_6H_5$, —$SO_2NH_2$, CN or phenyl,

X, if n = 1, is group of the formula —CO—$(NH)_a$—Y or

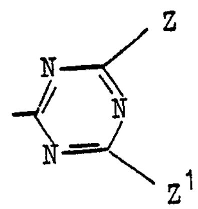

if n = 2, is a group of the formula —CO—, —CO—$(NH)_a$—Y'—$(NH)_a$—CO—

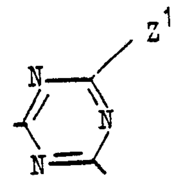

and if n = 3, is a group of the formula

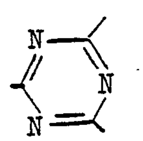

a is 0 or 1, Y is alkyl, alkoxy, phenyl, chlorophenyl or alkylphenyl, Y' is alkylene, phenylene, chlorophenylene, alkylphenylene, bis-(4-cyclohex-1-yl)-methane or, if a = 0, is a direct bond, Z is chlorine, hydroxyethylamino or bis-hydroxyethylamino, and $Z^1$ is chlorine, hydroxyethylamino, bis-hydroxyethylamino, and $Z^1$ is chlorine, hydroxyethylamino, bis-hydroxyethylamino, dialkylaminoalkylamino, trialkylammoniumalkylamino, sulfoethylamino, N-alkyl-N-sulfoethylamino, piperazinyl, N-hydroxyethylpiperazinyl, N-methyl-N-hydroxyethylpiperazinyl, phenylamino, sulfophenylamino, disulfophenylamino, alkylphenylamino or sulfatoethylsulfonylphenylamino.

3. A process for the preparation of a perfluoroalkyl ester of an anthranilic acid, of the formula (1) as claimed in claim 1, which comprises reacting an isatoic anhydride (2) with a perfluoroalkyl alcohol or perfluoroalkoxyperfluoroalkyl alcohol, preferably in the presence of an alkaline catalyst, to give the perfluoroalkylanthranilic acid ester intermediate product (3)

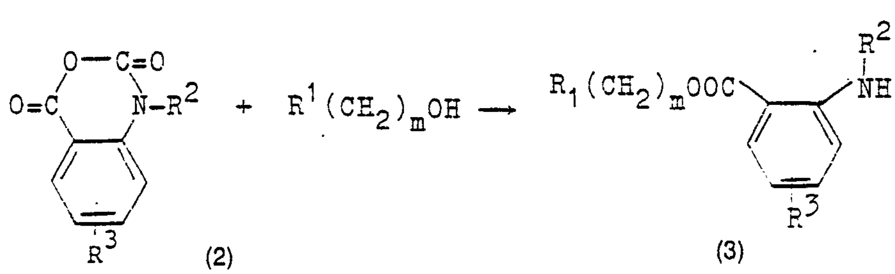

(2) (3)

an then

a) acylating it with an acid halide of an aliphatic or aromatic monocarboxylic acid or polycarboxylic acid or

b) condensing with a halogen-active heterocyclic compound, preferably cyanuric chloride and optionally replacing the halogen atoms in the condensation product by the radicals of optionally substituted aliphatic or aromatic amines or by

c) reacting further with an aliphatic or aromatic monoisocyanate or diisocyanate.

4. Use of the perfluoroalkyl-anthranilic acid ester of the formula (1) for making textile material oil an water-repellant.

**Revendications**

1. Esters perfluoroalkyliques d'acides anthraniliques répondant à la formule 1 :

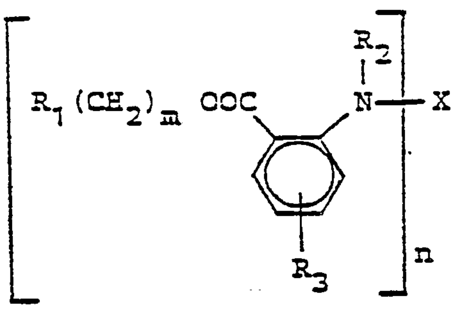

(1)

dans laquelle :

$R_1$ représente un radical perfluoralkyle ou un radical perfluoralcoxy-perfluoralkyle contenant chacun au total de 2 à 20 atomes de carbone, de préférence de 4 à 14,

$R_2$ représente l'hydrogène, un alkyle en $C_1$-$C_4$ linéaire ou ramifié ou un phényle,

$R_3$ représente l'hydrogène ou un ou plusieurs radicaux non chromophores pris dans l'ensemble constitué par le fluor, le chlore, le brome, les alkyles inférieurs, les alcoxy inférieurs, les acylamino, le carboxy, les carboxy modifiés fonctionnellement, le sulfo, les sulfo modifiés fonctionnellement et le phényle,

X représente le radical d'un acide mono- ou polycarboxylique aliphatique ou aromatique, le radical d'un composé hétérocyclique à halogène actif, les atomes d'halogènes du composé hétérocyclique qui ne sont pas remplacés par le radical de l'anthranilate de perfluoroalkyle pouvant être remplacés par des radicaux d'amines aliphatiques ou aromatiques, un radical alkylaminocarbonyle ou un radical arylamino-carbonyle,

m représente un nombre entier de 1 à 6, de préférence de 1 à 4, et

n représente un nombre entier de 1 à 3.

2. Esters perfluoroalkyliques d'acides anthraniliques de formule 1 selon la revendication 1, dans lesquels :

$R_1$ représente un radical de formule $C_lF_{2l+1}$—, un radical de formule $H(C_2F_4)_o$— ou un radical de formule $(CF_3)_2CFO(CF_2)_p$—,

l désigne un nombre égal à 6, à 8, à 10, à 12 ou à 14,

o désigne un nombre égal à 1, à 2, à 3 ou à 4,

p désigne un nombre entier de 2 à 8,

$R_2$ représente l'hydrogène ou un radical méthyle, éthyle ou phényle,

$R_3$ représente l'hydrogène ou un ou plusieurs radicaux pris dans l'ensemble constitué par le chlore et les radicaux méthyle, méthoxy, —COOH, —$COOCH_3$, —$SO_3H$, —$SO_2OC_6H_5$, —$SO_2NH_2$, —CN et phényle,

X représente dans le cas où n est égal à 1, un radical répondant à l'une des formules suivantes : —CO—$(NH)_a$—Y et

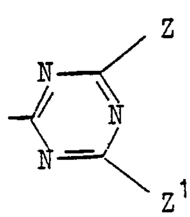

dans le cas où n est égal à 2, un radical de formule —CO—, —CO—$(NH)_a$—Y'—$(NH)_a$—CO— ou

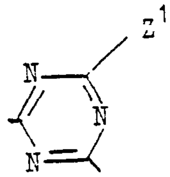

et dans le cas où n est égal à 3, un radical de formule :

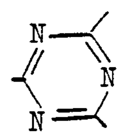

a est égal à 0 ou à 1, Y représente un radical alkyle, alcoxy, phényle, chlorophényle ou alkylphényle, Y′ représente un radical alkylène, phénylène, chlorophénylène, alkylphénylène ou (dicyclohexylméthane)-diyle-4,4′ ou, lorsque a est égal à 0, une liaison directe, Z représente le chlore ou un radical hydroxyéthylamino ou bis-hydroxyéthylamino, $Z^1$ représente le chlore ou un radical hydroxyéthylamino, bis-hydroxyéthylamino, di-alkylaminoalkylamino, trialkylammonium-alkylamino, sulfo-éthylamino, N-alkyl-N-sulfoéthylamino, pipérazinyle, N-hydroxyéthyl-pipérazinyle, N-méthyl-N-hydroxyéthyl-pipérazinyle, phénylamino, sulfophénylamino, di-sulfophénylamino, alkylphénylamino ou (sulfatoéthylsulfonyl)-phénylamino.

3. Procédé de préparation des esters perfluoroalkyliques d'acides anthraniliques de formule 1 selon la revendication 1, procédé caractérisé en ce qu'on fait réagir des anhydrides isatoïques (2) avec des perfluoralkyl-alcools ou des perfluoralcoxy-perfluoralkyl-alcools, de préférence en présence de catalyseurs alcalins, de manière à obtenir, comme corps intermédiaires, les esters perfluoroalkyliques d'acides anthraniliques (3) :

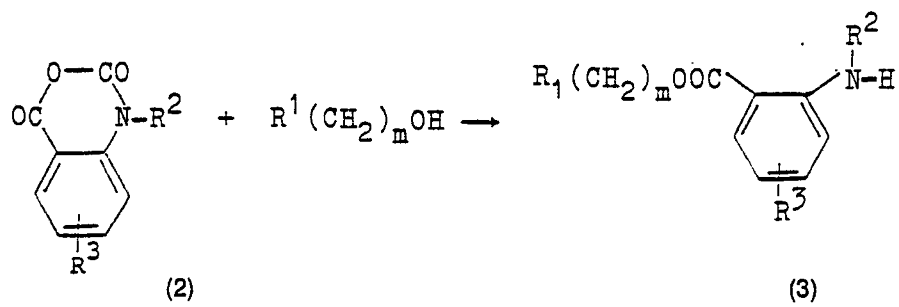

(2) (3)

et

a) on acyle ces derniers avec des halogénures d'acides monocarboxyliques ou polycarboxyliques aliphatiques ou aromatiques, ou

b) on les condense avec des composés hétérocycliques à halogène actif, de préférence avec le chlorure de cyanuryle, et, dans les produits de condensation, on échange éventuellement les atomes d'halogènes contre des radicaux d'amines aliphatiques ou aromatiques éventuellement substituées, ou

c) on les fait réagir avec des mono-isocyanates ou des di-isocyanates aliphatiques ou aromatiques.

4. Application des esters perfluoralkyliques d'acides anthraniliques de formule 1 selon la revendication 1 pour l'ennoblissement oléofuge et hydrofuge de matières textiles.